# EUROPEAN PATENT APPLICATION

(11) **EP 4 382 097 A1**
(43) Date of publication of application: **12.06.2024**
(21) Application number: 23211324.1
(22) Date of filing: 21.11.2023
(51) Int. Cl.: A61K 9/20, A61K 31/277, A61P 37/06

(54) **A TABLET COMPRISING TERIFLUNOMIDE**

(30) Priority: 25.11.2022 TR 202201784
(71) Applicant: SANOVEL ILAC SANAYI VE TICARET A.S., Istanbul 34460 (TR)
(72) Inventor: SUNEL, Fatih, Istanbul (TR); BILGEHAN ATAK, Fadime, Istanbul (TR); ATAMAN, Seval, Istanbul (TR); ERIS, Duygu, Istanbul (TR); YAZICI, Ozlem, Istanbul (TR); SARP, Onder, Istanbul (TR)
(74) Representative: Sevinç, Erkan

(57) **Abstract**

The present invention relates to a film coated tablet comprising teriflunomide, wherein the tablet comprising calcium stearate as lubricant and teriflunomide has a d (0.5) particle between 1 µm and 18 µm. Further the present invention discloses a method for the preparation of said tablet.

## Description

### Field of the Invention

The present invention relates to a film coated tablet comprising teriflunomide, wherein the tablet comprising calcium stearate as lubricant and teriflunomide has a d (0.5) particle between 1 µm and 18 µm. Further the present invention discloses a method for the preparation of said tablet.

### Background of the Invention

Multiple Sclerosis (MS) is a demyelinating disease characterized by damage to insulating ends of neurons in the brain and spinal cord. This damage impairs the communication between the components of central nervous system and leads to a series of signs and symptoms including physical, mental, and sometimes, psychiatric problems. Specific symptoms include diplopia, blindness in one eye, muscle weakness, sensory impairment or coordination problems. MS presents with new symptoms either in isolated attacks (in relapsing-remitting forms) or over time (progressively). Symptoms may completely resolve in between attacks. In addition, the likelihood of having permanent neurological damage is high especially as the disease progresses.

Teriflunomide is a pyrimidine synthesis inhibitor. Teriflunomide acts via the mechanism of a dihydroorotate dehydrogenase inhibitor and it is an orally available immunomodulator used in the treatment of relapsing-remitting multiple sclerosis. Chemical name of teriflunomide is (Z)-2-cyano-3-hydroxy-N-[4-(trifluoromethyl)phenyl]but-2enamide and its chemical structure is shown in Formula I.

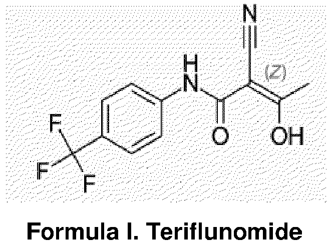

Teriflunomide inhibits rapidly dividing cells including activated T cells that are believed to drive the disease process in MS and it may decrease the risk of infection thanks to its more limited effects on the immune system compared to chemotherapeutic agents.

The document EP0527736B1 is the first molecule patent that describes teriflunomide in the prior art. It mentions the use of teriflunomide in the prophylaxis and/or treatment of rheumatic diseases and the possibility to administer the pharmaceuticals of the invention orally, topically, rectally and parenterally, if required.

US8802735 application discloses a solid pharmaceutical composition of Teriflunomide. The patent teaches that teriflunomide tablets cannot be made with colloidal silicon dioxide as it leads to an unstable product. The patent teaches that teriflunomide tablets without colloidal silicon dioxide display significantly reduced formation of degradants, compared to Teriflunomide tablets containing colloidal silicon dioxide.

In the prior art, compositions comprising teriflunomide show a slightly less degradation of teriflunomide but cannot shed a light on stability and solubility problems. According to the prior art, as provided above, there is still a need for stable and highly soluble teriflunomide formulations.

### Detailed Description of the Invention

The main object of the present invention is to obtain a film coated tablet comprising teriflunomide that eliminate the above-mentioned problems and provide additional advantages to the relevant prior art.

Another object of the present invention is to obtain a film coated tablet of teriflunomide with high stability, flowability, hardness and the desired solubility profile.

Teriflunomide is a Biopharmaceutics Classification System Class (BCS) II drug with low solubility and high permeability. We have overcome the dissolution problems we encountered while developing tablets by using low particle size. However, this caused some problems during production and printing, such as sticking to staples, and loss of active material. We have tried many things to overcome this. We found that the use of teriflunomide low particle size together with calcium stearate as a lubricant provides the desired dissolution and stability.

We have found that teriflunomide having the following particle sizes is very important for formulation. Especially, it positively affects the dissolution properties and powder blend uniformity. The obtained tablets have the desired dissolution profile. The powder is more homogeneous. The content uniformity of the tablets obtained from the more homogeneous powder is more ideal. Therefore, it provides better bioavailability.

Teriflunomide can stick to the punch during manufacturing. This can cause problems, for example; tablet shape deformations, variations in content uniformity, during manufacturing. We found that calcium stearate is a very good lubricant for tablet comprising teriflunomide.

By using calcium stearate, we have overcome the fluidity problem that will occur due to the small particle size. Especially, in the present invention, using calcium stearate is provided the desired stability. Using lubricant especially, calcium stearate provides to overcome compressibility and stick problems of the active agent. Also, calcium stearate show a substantial improvement in hardness and an exceptional improvement in friability.

As used here in, 'particle size' means the cumulative volume size distribution as tested by any conventionally accepted method such as the laser diffraction method (i.e. malvern analysis). The term d (0.9) means the size at which %90 by volume of the particles are finer and The term d (0.5) means the size at which %50 by volume of the particles are finer.

According to this embodiment of the present invention, a film coated tablet comprising teriflunomide, wherein the tablet comprising calcium stearate as lubricant and teriflunomide has a d (0.5) particle between 1 µm and 18 µm.

According to this embodiment of the present invention, teriflunomide has a d (0.5) particle size less than 18 µm, preferably less than 13 µm, more preferably less than 8 µm. Preferably, it is between 1 µm and 18 µm.

According to this embodiment of the present invention, teriflunomide has a d (0.9) particle size less than 20 µm, preferably less than 15 µm, more preferably less than 10 µm. Preferably, it is between 1 µm and 20 µm.

According to this embodiment of the present invention, the amount of teriflunomide is between 2.0% and 15.0% by weight in the total tablet.

According to this embodiment of the present invention, the amount of calcium stearate is between 0.1% and 2.0% by weight of the total formulation.

In general terms, excipients provided in a formulation may positively or negatively influence the physicochemical and pharmacokinetic properties, e.g. the solubility, degradation, absorption, bioavailability of an active agent. For this reason, the excipients which accompany an active agent have to be selected in a careful and conscious manner while a formulation is developed. The formulations should have no physicochemical incompatibility between the active agents and the excipients.

According to this embodiment of the present invention, at least one pharmaceutically acceptable excipient is selected from the group comprising fillers, binders, disintegrants, lubricants or mixtures thereof.

Suitable fillers are selected from group comprising lactose monohydrate, ammonium alginate, calcium carbonate, calcium phosphate, calcium phosphate dehydrate, neutral pellets, calcium sulfate, cellulose, cellulose acetate, dextrose, erythritol, ethyl cellulose, fructose, glyceryl palmitostearate, lactose, mannitol, magnesium carbonate, magnesium oxide, maltodextrin, maltose, polydextrose, polymethacrylates, polyvinylpyrrolidone, simethicone, sodium alginate, sodium chloride, sorbitol, starch, sucrose, sugar spheres, sulfobutylether beta-cyclodextrin or mixtures thereof.

According to this embodiment of the present invention, the amount of fillers is between 25.0% and 40.0% by weight of the total formulation. Preferably, the amount of fillers is between 29.0% and 37.0% by weight of the total formulation.

According to this embodiment of the present invention, the filler is lactose monohydrate.

Suitable binders are selected from the group comprising corn starch, hydroxypropyl cellulose, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

According to this embodiment of the present invention, the amount of binders is between 1.0% and 40.0% by weight of the total formulation. Preferably, the amount of binders is between 29.0% and 35.0% by weight of the total formulation.

According to this embodiment of the present invention, the binder is corn starch or hydroxypropyl cellulose or mixtures thereof.

According to this embodiment of the present invention, the amount of corn starch is between 20.0% and 35.0% by weight of the total formulation.

According to this embodiment of the present invention, the amount of hydroxypropyl cellulose is between 1.0% and 5.0% by weight of the total formulation.

According to this embodiment of the present invention, at least two binder is used in the formulation.

Suitable disintegrants are selected from the group comprising sodium starch glycolate, microcrystalline cellulose, croscarmellose sodium, crospovidone, alginates, magnesium aluminum silica, sodium carboxymethyl cellulose, calcium silicate, carboxymethyl cellulose, calcium carboxymethyl cellulose, low substituted hydroxypropyl cellulose, polyacryline potassium, poloxamer, sodium alginate, sodium glycine carbonate, sodium dodecyl sulfate or mixtures thereof.

According to this embodiment of the present invention, the amount of disintegrants is between 5.0% and 40.0% by weight of the total formulation. Preferably, the amount of disintegrants is between 20.0% and 30.0% by weight of the total formulation.

In the film coated tablet, the ratio of disintegrant to a teriflunomide is in the range of between 1:4 and 4:1, preferably between 1:3 and 3:1. The ratio provide the desired dissolution profile.

According to this embodiment of the invention, the disintegrant is sodium starch glycolate or microcrystalline cellulose or mixtures thereof.

According to this embodiment of the present invention, the amount of sodium starch glycolate is between 5.0% and 15.0% by weight of the total formulation.

According to this embodiment of the present invention, the amount of microcrystalline cellulose is between 5.0% and 25.0% by weight of the total formulation.

According to this embodiment of the invention, at least two disintegrant is used in the formulation.

Suitable lubricants are selected from the group comprising talc, zinc stearate, sodium chlorate, sodium oleate, sodium acetate, sodium benzoate, polyethylene glycol, glyceryl palmito sulphate, sodium stearyl fumarate, sodium lauryl sulphate or mixtures thereof.

According to this embodiment of the present invention, the amount of lubricants is between 0.1% and 8.0% by weight of the total formulation. Preferably, the amount of lubricants is between 0.5% and 5.0% by weight of the total formulation.

According to this embodiment of the invention, the lubricant is talc.

According to this embodiment of the present invention, the amount of talc is between 1.0% and 6.0% by weight of the total formulation.

According to this embodiment of the invention, at least two lubricant is used in the formulation.

In the present invention, the tablet comprises film coating to protect the composition against the moisture and light to maintain the stability. Suitable film coating agents are selected from the group comprising hydroxypropylmethylcellulose, polyethylene glycol (PEG), talc, titanium dioxide, polyvinyl alcohol (PVA), polyvinyl alcohol-polyethylene glycol copolymers, polyvinylprolidone, lecithin, polyvinylprolidone-vinyl acetate copolymer (PVP-VA), pigments, dyes, iron oxide or mixtures thereof.

According to one embodiment of the invention, the film coated tablet has scored. In this way, it can be easily divided into two for use. Improved hardness and strength of the tablet is significantly essential for this invention since the composition is preferably in the form of a scored tablet which is supposed to be split in half and this kind of tablets should have the strength not to crumble to more than two pieces.

The film coated tablet of the present invention can be prepared, using standard techniques and manufacturing processes well known in the art, such as direct compression, wet or dry granulation, hot melt granulation, hot melt extrusion, fluidized bed granulation, extrusion/spheronization, slugging, spray drying and solvent evaporation.

It has surprisingly been found that the film coated tablet of excellent uniformity and showing improved compressibility can be obtained when teriflunomide and all excipients (except for lubricants) are formulated together in the wet granulation process. Wet granulation process efficiently counteracts segregation, so it can achieve good dissolution and disintegration properties. Solvents are used in the process.

According to this embodiment of the invention, the film coated tablet is prepared with using wet granulation.

Suitable solvents are selected from a group comprising pure water, ethyl alcohol, glycerin, sorbitol, polyethylene glycol, propylene glycol, isopropyl alcohol, or mixtures thereof, preferably granulation solution is pure water.

According to this embodiment of the invention, a process for preparing of a film coated tablet comprising teriflunomide, wherein comprising the following steps;
a) Mixing teriflunomide, lactose monohydrate, corn starch, a half of microcrystalline cellulose, a half of sodium starch glycolate and hydroxypropyl cellulose,
b) Granulating the mixture with pure water by using high shear granulator,
c) Drying the mixture in fluidized bed,
d) Sieving the mixture,
e) Sieving talc, the remaining part of microcrystalline cellulose and the remaining part of sodium starch glycolate and then adding into step (d) and mixing,
f) Adding calcium stearate and mixing,
g) Compressing the mixture to form tablets,
h) Coating the tablets with film coating comprising film coating agents.

### Example 1: A film coated tablet comprising teriflunomide

| **Ingredients** | **Amount (% by weight of the total)** |
|---|---|
| Teriflunomide | 2.0 - 15.0 |
| Lactose monohydrate | 25.0 - 40.0 |
| Corn starch | 20.0 - 35.0 |
| Hydroxypropyl cellulose LF | 1.0 - 5.0 |
| Microcrystalline cellulose PH 101 | 5.0 - 25.0 |
| Sodium starch glycolate | 5.0 - 15.0 |
| Talc | 1.0 - 6.0 |
| Calcium stearate | 0.1 - 2.0 |
| Film coating agents | 1.0 - 6.0 |
| **Total composition** | **100** |

### Example 2: A film coated tablet comprising teriflunomide

| **Ingredients** | **Amount (% by weight of the total)** |
|---|---|
| Teriflunomide | 9 |
| Lactose monohydrate | 30.6 |
| Corn starch | 27.7 |
| Hydroxypropyl cellulose | 2.23 |
| Microcrystalline cellulose | 13.5 |
| Sodium starch glycolate | 9.68 |
| Talc | 3.5 |
| Calcium stearate | 0.3 |
| Film coating agents | 3.22 |
| ***Total composition*** | **100** |

**A process for example 1 or 2;**
a) Mixing teriflunomide, lactose monohydrate, corn starch, a half of microcrystalline cellulose, a half of sodium starch glycolate and hydroxypropyl cellulose,
b) Granulating the mixture with pure water,
c) Drying the mixture in fluidized bed at 45°C,
d) Sieving the mixture,
e) Sieving talc, the remaining part of microcrystalline cellulose and the remaining part of sodium starch glycolate and then adding into step (d) and mixing for 15 minutes,
f) Adding calcium stearate and mixing for 3 minutes,
g) Compressing the mixture to form tablets,
h) Coating the tablets with film coating comprising film coating agents.

### Film coating agents

| |
|---|
| Hydroxypropyl methylcellulose |
| Titanium dioxide |
| Macrogol/Polyethylene glycol |
| FD&C BLUE #2/INDIGO CARMINE AL |

## Claims

1. A film coated tablet comprising teriflunomide, wherein the tablet comprising calcium stearate as lubricant and teriflunomide has a d (0.5) particle between 1 µm and 18 µm.

2. The film coated tablet according to claim 1, wherein teriflunomide has a d (0.5) particle size less than 18 µm, preferably less than 13 µm, more preferably less than 8 µm.

3. The film coated tablet according to claim 1, wherein teriflunomide has a d (0.9) particle size less than 20 µm, preferably less than 15 µm, more preferably less than 10 µm.

4. The film coated tablet according to claim 1, wherein the tablet further comprising at least one pharmaceutically acceptable excipient which is selected from the group comprising fillers, binders, disintegrants, lubricants or mixtures thereof.

5. The film coated tablet according to claim 4, wherein fillers are selected from group comprising lactose monohydrate, ammonium alginate, calcium carbonate, calcium phosphate, calcium phosphate dehydrate, neutral pellets, calcium sulfate, cellulose, cellulose acetate, dextrose, erythritol, ethyl cellulose, fructose, glyceryl palmitostearate, lactose, mannitol, magnesium carbonate, magnesium oxide, maltodextrin, maltose, polydextrose, polymethacrylates, polyvinylpyrrolidone, simethicone, sodium alginate, sodium chloride, sorbitol, starch, sucrose, sugar spheres, sulfobutylether beta-cyclodextrin or mixtures thereof.

6. The film coated tablet according to claim 4, wherein binders are selected from the group comprising corn starch, hydroxypropyl cellulose, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

7. The film coated tablet according to claim 4, wherein disintegrants are selected from the group comprising sodium starch glycolate, microcrystalline cellulose, croscarmellose sodium, crospovidone, alginates, magnesium aluminum silica, sodium carboxymethyl cellulose, calcium silicate, carboxymethyl cellulose, calcium carboxymethyl cellulose, low substituted hydroxypropyl cellulose, polyacryline potassium, poloxamer, sodium alginate, sodium glycine carbonate, sodium dodecyl sulfate or mixtures thereof.

8. The film coated tablet according to claim 7, wherein the ratio of disintegrant to a teriflunomide is in the range of between 1:4 and 4:1, preferably between 1:3 and 3:1.

9. The film coated tablet according to claim 4, wherein lubricants are selected from the group comprising talc, zinc stearate, sodium chlorate, sodium oleate, sodium acetate, sodium benzoate, polyethylene glycol, glyceryl palmito sulphate, sodium stearyl fumarate, sodium lauryl sulphate or mixtures thereof.

10. The film coated tablet according to claim 9, wherein the lubricant is talc.

11. The film coated tablet according to claim 1, wherein the film coated tablet is prepared with using wet granulation.

12. The film coated tablet according to claim 11, wherein solvents are selected from a group comprising pure water, ethyl alcohol, glycerin, sorbitol, polyethylene glycol, propylene glycol, isopropyl alcohol, or mixtures thereof.

13. The film coated tablet according to claim 11, wherein the tablet has scored.

14. A process for preparing of a film coated tablet comprising teriflunomide, wherein comprising the following steps;
a) Mixing teriflunomide, lactose monohydrate, corn starch, a half of microcrystalline cellulose, a half of sodium starch glycolate and hydroxypropyl cellulose,
b) Granulating the mixture with pure water,
c) Drying the mixture in fluidized bed,
d) Sieving the mixture,
e) Sieving talc, the remaining part of microcrystalline cellulose and the remaining part of sodium starch glycolate and then adding into step (d) and mixing,
f) Adding calcium stearate and mixing,
g) Compressing the mixture to form tablets,
h) Coating the tablets with film coating comprising film coating agents.
